Europäisches Patentamt

European Patent Office

Office européen des brevets

(11) Veröffentlichungsnummer: **0 290 901 B1**

(12) **EUROPÄISCHE PATENTSCHRIFT**

(45) Veröffentlichungstag der Patentschrift: **24.03.93**

(51) Int. Cl.[5]: **C07C 62/14**, B03D 1/00

(21) Anmeldenummer: **88107008.0**

(22) Anmeldetag: **02.05.88**

(54) **Bicyclische Decandicarbonsäuren, Verfahren zu ihrer Herstellung und ihre Verwendung als Flotationshilfsmittel.**

(30) Priorität: **11.05.87 DE 3715613**

(43) Veröffentlichungstag der Anmeldung:
**17.11.88 Patentblatt 88/46**

(45) Bekanntmachung des Hinweises auf die
Patenterteilung:
**24.03.93 Patentblatt 93/12**

(84) Benannte Vertragsstaaten:
**DE**

(56) Entgegenhaltungen:

TETRAHEDRON LETTERS, Nr. 42, 1974, Seiten
3685-3688, Pergamon Press; K. FURUICHI et
al.: "Total synthesis of (+)-trans-sweroside
aglucone o-methyl ether"

CHEMICAL ABSTRACTS, Band 102, Nr. 13, 1.
April 1985, Seite 691, Zusammenfassung Nr.
113268m, Columbus, Ohio, US; & JP-A-59 170
086

CHEMICAL ABSTRACTS, Band 102, Nr. 13, 1.
April 1985, Seite 693, Zusammenfassung Nr.
113295t, Columbus, Ohio, US; & JP-A-59 170
087

(73) Patentinhaber: **Henkel Kommanditgesellschaft
auf Aktien
Postfach 1100 Henkelstrasse 67
W-4000 Düsseldorf-Holthausen(DE)**

(72) Erfinder: **Eierdanz, Horst, Dr.
Verbindungsstrasse 5
W-4010 Hilden(DE)**
Erfinder: **Schulz, Paul, Dr.
Auf dem Scheidt 35
W-5600 Wuppertal 1(DE)**
Erfinder: **von Rybinski, Wolfgang, Dr.
Leinenweberweg 12
W-4000 Düsseldorf 13(DE)**
Erfinder: **Köster, Rita
Taubenstrasse 7
W-4000 Düsseldorf 30(DE)**

EP 0 290 901 B1

EP 0 290 901 B1

**Beschreibung**

Die Erfindung betrifft bicyclische Decandicarbonsäuren erhältlich durch
a) säurekatalysierte Umsetzung von Dicyclopentadien mit einer Verbindung der Formel (I),

R-OH      (I)

in der R aus der Gruppe der
a) linearen oder verzweigten, gesättigten Alkylreste mit 1 bis 22 Kohlenstoffatomen,
b) linearen oder verzweigten, gesättigten Alkoxycarbonylreste mit 2 bis 34 Kohlenstoffatomen und
c) Reste polyalkoxylierter Fettalkohole der Formel II

$R^1O-(CH-CHR^2-O)_n-CH_2-CHR^2-$      (II)

in der $R^1$ ein gesättigter Fettalkoholrest mit 2 bis 22 Kohlenstoffatomen, $R^2$ Wasserstoff und/oder Methyl und n eine Zahl von 0 bis 24 bedeuten,
ausgewählt ist, und
b) oxidative Spaltung der erhaltenen bicyclischen Verbindung an der Doppelbindung des Fünfrings,
ein Verfahren an ihrer Herstellung sowie ihre Verwendung als Flotationshilfsmittel.

Dicarbonsäuren auf Basis von Fettstoffen, zum Beispiel die sogenannten Dimerfettsäuren, sind wichtige Verbindungen mit einer Vielzahl von Anwendungsmöglichkeiten, vgl. Polymers Paint Colour Journal, Europ. Supplement, 1985, 115. Die günstigen Eigenschaften dieser Stoffklasse beruhen im wesentlichen auf dem Vorliegen langer Alkylketten. Dicarbonsäuren mit mehr als 12 Kohlenstoffatomen lassen sich jedoch nur durch wenige Reaktionen darstellen.

So führt die Dimerisierung von ungesättigten Fettsäuren zu den obengenannten Dimerfettsäuren mit 36 Kohlenstoffatomen. Die Reaktion von Fettsäuren mit Acrylsäure führt zu einer $C_{21}$-Dicarbonsäure, vgl. IAOCS 57, 219 (1975). Die Hydrocarboxylierung von ungesättigten Fettsäuren ergibt $C_{19}$-Dicarbonsäuren, vgl. Fette, Seifen, Anstrichmittel 87, 400 (1985).

Es wurde nun gefunden, daß sich durch eine einfache Reaktion eine Gruppe neuer bicyclischer Dicarbonsäuren auf Basis von Fettstoffen aufbauen lassen, die interessante Eigenschaften aufweisen, z.B. als Zwischenprodukte oder als Flotationshilfsmittel, insbesondere als Sammler für die Flotation nichtsulfidischer Erze.

Ausgangsmaterial für die Herstellung der erfindungsgemäßen bicyclischen Decandicarbonsäuren sind Dicyclopentadien und lineare oder verzweigte, gesättigte Alkohole mit 1 bis 22 Kohlenstoffatomen, z. B. Methanol, Ethanol, n-Propanol, i-Propanol, n-Butanol und dessen Isomere, 2-Ethylhexanol, verzweigte Octanole sowie Fettalkohole mit 8 bis 18 Kohlenstoffatomen, auch in Form ihrer technischen Gemische, wie sie bei der Fettalkoholsynthese anfallen. Man erhält bicyclische Decandicarbonsäuren, die einen Ethersubstituenten tragen.

Alternativ können anstelle der vorgenannten Alkohole auch lineare oder verzweigte, gesättigte Fettsäuren mit Dicyclopentadien umgesetzt werden, z. B. Essig-, Propion-, Butter-, Valerian-, Capron-, Önanth-, Capryl-, Pelargon-, Caprin-, Undecan-, Laurin-, Tridecan-, Myristin-, Pentadecan-, Palmitin-, Margarin-, Stearin-, Nonadecan-, Arachin-, Behen-, Lignocerin-, Cerotin-, Melissin- und Montansäure. Es können auch Fettsäuregemische eingesetzt werden, wie sie bei der Spaltung tierischer oder pflanzlicher Fette erhalten werden. Es lassen sich auf diese Weise im Sinne der Erfindung bicyclische Decandicarbonsäuren herstellen, die mit einer Esterfunktion substituiert sind.

Alternativ können im Sinne der Erfindung bicyclische Decandicarbonsäuren auch ausgehend von Dicyclopentadien und polyalkoxylierten Fettalkoholen hergestellt werden; die Fettalkohole können die obigen Bedeutungen aufweisen, bevorzugt sind jedoch solche mit 8 bis 18 Kohlenstoffatomen, auch in Form ihrer technischen Gemische, wie sie aus tierischen oder pflanzlichen Fetten erhalten werden können. Diese Fettalkohole werden in Form ihrer ethoxylierten und/oder propoxylierten Derivate eingesetzt, wobei sie formal mit 1 bis 25, vorzugsweise mit 2 bis 10 Ethylenoxid- und/oder Propylenoxideinheiten umgesetzt sind. Ethylenoxid- und Propylenoxideinheiten können dabei eine random- oder block-Verteilung aufweisen.

Die Erfindung betrifft weiterhin ein Verfahren zur Herstellung bicyclischer Decandicarbonsäuren, das dadurch gekennzeichnet ist, daß man Dicyclopentadien mit einer Verbindung der allgemeinen Formel R-OH, in der R wie oben definiert ist, umsetzt und die erhaltene bicyclische Verbindung an der Doppelbindung des Fünfringes oxidativ spaltet.

Wie bereits ausgeführt, erfolgt die Umsetzung von Dicyclopentadien mit den Verbindungen der Formel R-OH unter Säurekatalyse, vgl. US-PS 2 394 582, in an sich bekannter Weise.

2

Die oxidative Spaltung der Doppelbindung des Fünfringes kann nach zahlreichen an sich bekannten Verfahren erfolgen, z. B. durch Ozonolyse (K. Griesbaum, Brennstoff-Chemie 50, 212 (1969)), durch Spaltung mit Permanganat oder Perjodat (Can. J. Chem. 33, 1714 (1955)), durch Umsetzung mit Persäuren in Gegenwart von Rutheniumtrichlorid (DE-A 21 06 307), durch Umsetzung mit Rutheniumoxid, Acetaldehyd und Sauerstoff (EP-A 0 021 118), durch Oxidation mit Sauerstoff in Gegenwart von Acetaldehyd und Verbindungen von V, Mo, W oder Os (US-PS 3 701 804), durch Umsetzung mit Rutheniumtetroxid und Natriumhypochlorit (JAOCS 1977, 870A), durch Umsetzung von Rutheniumtrichlorid und Natriumhypochlorit (Chem. Comm. 21, 1420 (1970)), durch Umsetzung mit Salpetersäure und Ammoniumvanadat (US-PS 2 323 861, GB-PS 1 068 905, FR-PS 981 609), durch Umsetzung mit Salpetersäure (US-PS 2 265 601).

Die Spaltung der Doppelbindung unter Bildung von Dicarbonsäuren ist auch möglich, indem man nach üblichen Verfahren (F.D. Gunstone, Hydroxylation Methods, Advances in Organic Chemistry, Band I, Interscience Publishers, S. 103 ff, 1960) das Diol herstellt und dieses zur Carbonsäure spaltet, vgl. auch DE-OS 20 52 815, 21 06 913 (Umsetzung mit Sauerstoff, Peressigsäure in Gegenwart von Kobalt(II)acetat), sowie DE-OS 20 35 558 (Umsetzung mit Peressigsäure in Gegenwart von Ni-, Fe-, Pd-, Mn- oder Co-Verbindungen).

Die Erfindung betrifft weiterhin die Verwendung der bicyclischen Decandicarbonsäuren als Flotationshilfsmittel.

Die Erfindung wird im folgenden anhand bevorzugter Ausführungsbeispiele näher erläutert.

Arbeitsvorschriften für die Herstellung von Ethern und Estern des Dicyclopentadiens (Ausgangsmaterial)

Die Synthesen wurden analog US-PS 2 394 582 bzw. US-PS 2 395 452 durchgeführt.

A) Umsetzungsprodukt aus Dicyclopentadien und Laurylalkohol

132 g Dicyclopentadien, 186 g Laurylalkohol und 30 g Borfluoridetherat wurden unter Rühren vier Stunden lang auf ca. 100°C erhitzt. Die Reaktionsmischung wurde zunächst mit heißem Wasser, danach mit Natriumcarbonatlösung und erneut mit Wasser gewaschen, getrocknet und im Vakuum destilliert. Das gewünschte Produkt ging bei 185°C/2 mbar als schwach gelbes Öl über.

Nach demselben Verfahren wurden die Umsetzungsprodukte aus Dicyclopentadien und 2-Ethylhexanol (Sdp. 115 - 120°C/1 mbar) und Ethanol (Sdp. 109°C/18 mbar) erhalten. In analoger Weise, jedoch ohne Destillation des Reaktionsproduktes, wurden Umsetzungsprodukte des Dicyclopentadiens mit Stearylalkohol, Isotridecylalkohol und einem Anlagerungsprodukt von 3 Mol Ethylenoxid an ein Fettalkoholgemisch der Kettenlängen $C_{12}$-$C_{18}$ erhalten.

b) Umsetzungsprodukt aus Dicyclopentadien und Essigsäure

100 g Dicyclopentadien, 250 g Essigsäure, 4 g konzentrierte Schwefelsäure und 6 g Wasser wurden unter Rühren 5 Stunden lang auf 70°C erhitzt. Danach wurde die Reaktionsmischung unter Rühren mit Wasser versetzt. Die organische Phase wurde abgetrennt, mit Natriumcarbonatlösung und mit Wasser gewaschen, getrocknet und im Vakuum destilliert. Das gewünschte Produkt ging bei 95 - 100°C/1 mbar als klare Flüssigkeit über.

In analoger Weise wurde ein Umsetzungsprodukt aus Dicyclopentadien und Laurinsäure, Sdp. 170 - 210°C/0,1 mbar erhalten.

Beispiel 1

In eine Edelstahl-Kolonne (3 m x 40 mm) mit Mellapack-Füllung wurden von oben nach unten im Gleichstrom kontinuierlich eingespeist:

5000 g/h einer 20 %-igen Lösung des bicyclischen Ethers erhalten nach obiger Arbeitsvorschift a) in Caprylsäure;

1000 g/h Wasser, das in einem Statikmischer der Olefinlösung kurz vor Eintritt in die Kolonne zugemischt wurde,

8 m³/h ozonisierte Luft (ca. 160 g Ozon/h, d.h. ein 10 %-iger Überschuß, bezogen auf das Olefin).

Die Reaktionsenthalpie wurde durch Wasserverdunstung im Trägergasstrom abgeführt; die Ozonolysetemperatur betrug stationär ca. 36°C am Kolonneneintritt und ca. 20°C am Austritt.

Die Ozonidlösung wurde von den Resten der wäßrigen Phase abgetrennt (ca. 250 g/h) und ohne Zwischenlagerung aufgearbeitet.

Die Aufarbeitung erfolgte diskontinuierlich. Die Lösung wurde in einem gläsernen oder emaillierten Reaktionsapparat unter intensiver Rührung bei 80°C, unter Einleitung von bei 60°C mit Wasserdampf abgesättigter Luft (ca. 70 l/h/kg Reaktionsgemisch) 1,5 Stunden autokatalytisch oxidiert.

Anschließend wurden, unter Beibehaltung der Luftzufuhr, in 2 Stunden bei 80°C nach und nach 28,5 g 70 %-ige Wasserstoffperoxid-Lösung auf 1 kg Reaktionsgemisch zugetropft.

In der darauffolgenden Nachreaktionsphase wurde der Reaktorinhalt in 30 Minuten auf 100°C aufgeheizt und 1 Stunde unter Zugabe weiterer 28,5 g $H_2O_2$ (70 %) auf 1 kg organische Phase gerührt.

Die Lufteinleitung wurde unterbrochen. Die Temperatur wurde unter Ausschleusen eines hauptsächlich aus Wasser bestehenden Destillats in ca. 1 Stunde von 100 auf 130°C angehoben. Die Endtemperatur wurde bis zu einer Peroxidzahl der organischen Lösung von ca. 10 Einheiten bei 130°C konstant gehalten (0,5 bis 1 h). Die durchreagierte Lösung war lagerfähig.

Die Caprylsäure wurde bei 2 mbar (Wärmeträgertemperatur 165°C) am Dünnschichtverdampfer abgezogen, der Rückstand in heißem Eisessig aufgenommen und unter Rührung und Kühlung kristallisiert. Das Produkt wurde in Form kleiner farbloser Kristalle erhalten, Fp. 101 bis 108°C, Säurezahl 292. Durch Einengen der Mutterlaugen konnte weiteres Produkt erhalten werden. Gesamtausbeute 85 %.

Beispiel 2.

Zu 39,1 g (0,149 mol) des bicyclischen Ethers, erhalten nach obiger Arbeitsvorschrift a) in 200 ml Aceton wurden bei Umgebungstemperatur innerhalb von 15 min 60 g (0,38 mol) $KMnO_4$ gegeben; anschließend wurde 2 Stunden am Rückfluß gekocht, warm abgesaugt und aus dem Filterkuchen nach Spülen mit Aceton die Dicarbonsäure mit Aceton/Wasser in der Wärme ausgewaschen. Nach dem Ansäuern und Ausethern der Filtrate wird gewaschen, getrocknet und eingeengt.

Man erhielt 46 % der hochviskosen, dunkelgelben Dicarbonsäure mit einer Säurezahl von 312 entsprechend 90 % des theoretischen Wertes.

Die physikalischen Daten der vorstehend genannten Verbindungen der Beispiele 1 und 2 sind zusammen mit denjenigen weiterer hergestellter Verbindungen der Erfindung in der Tabelle 1 zusammengefaßt.

Die Verbindungen der Erfindung wurden hinsichtlich ihrer Eignung als Flotationshilfsmittel untersucht. Die Flotationsversuche wurden in einer modifizierten Hallimond-Röhre (Mikroflotationszelle) nach B. Dobias, Colloid & Polymer Sci. 259 (1981), Seite 775 bis 776, bei 23°C durchgeführt. Die einzelnen Versuche wurden mit jeweils 2 g Erz vorgenommen. Zur Herstellung der Trübe wurde destilliertes Wasser verwendet. Den Trüben wurde jeweils soviel Sammler zugegeben, daß eine Sammlermenge von 500 g/t vorhanden war. Die Konditionierzeit betrug jeweils 15 Minuten. Während der Flotation wurde ein Luftstrom mit einem Durchfluß von 4 ml/min durch die Trübe geleitet. Die Flotationsdauer betrug in allen Versuchen 2 min.

Die Ergebnisse der Flotationsversuche sind in der folgenden Tabelle II wiedergegeben. In der ersten Spalte sind die als Sammler verwendeten Verbindungen der Erfindung bezeichnet. In der zweiten Spalte ist das Gesamtausbringen, bezogen auf die gesamte Erzmenge, und in der dritten Spalte das Metallausbringen, bezogen auf die im Erz vorhandene $WO_3$-Gesamtmenge, verzeichnet. In den vierten bis sechsten Spalten sind die $WO_3$-, $CaO$- und $SiO_2$-Gehalte der jeweiligen Konzentrate wiedergegeben. Als Erz wurde ein Scheeliterz aus Osterreich mit der nachstehenden chemischen Zusammensetzung, bezogen auf die Hauptbestandteile, eingesetzt:

| | |
|---|---|
| $WO_3$ | 0,4 % |
| $CaO$ | 8,3 % |
| $SiO_2$ | 58,2 % |
| $Fe_2O_3$ | 7,8 % |
| $Al_2O_3$ | 12,5 % |
| $MgO$ | 6,9 % |

Die Flotationsaufgabe hatte die folgende Korngrößenverteilung:

## Tabelle 1

| Beispiele | | Säurezahl | phys. Daten |
|---|---|---|---|
| Nr. | R | | |
| 1 | $C_{12}H_{25}-$ | 292 | Fp. 101-108°C |
| 2 | $CH_3-(CH_2)_3-CH(C_2H_5)-CH_2-$ | 312 | dkl. gelbes Öl |
| 3 | $C_2H_5-$ | 403 | braun, glasartig |
| 4 | $C_{18}H_{37}-$ | 237 | Fp. 72-100°C |
| 5 | Isotridecyl- | 240 | dkl. braune Flüssigkeit |
| 6 | $C_{12}-C_{18}-$Fettalkoholrest + 3EO | 191 | dkl. braune Flüssigkeit |
| 7 | $CH_3CO-$ | 367 | Fp. 142-174,5°C |
| 8 | $C_{11}H_{23}CO-$ | 132 | viskose Flüssigkeit |

EP 0 290 901 B1

Tabelle 2

Flotationsversuche
Österreichisches Scheeliterz, Mikroflotationszelle

| Sammler Bsp. | Ausbringen gesamt (%) | Ausbringen $WO_3$ (%) | Konzentratgehalt (%) | | |
|---|---|---|---|---|---|
| | | | $WO_3$ | CaO | $SiO_2$ |
| 1 | 5,0 | 31 | 2,1 | 16,8 | 44,0 |
| 2 | 1,7 | 14 | 2,6 | 11,1 | 47,7 |
| 3 | 2,0 | 18 | 2,9 | 9,4 | 48,7 |
| 4 | 1,8 | 24 | 4,4 | 11,9 | 44,6 |
| 8 | 3,5 | 37 | 3,6 | 12,7 | 45,4 |
| 6 | 16,5 | 58 | 1,2 | 12,4 | 48,6 |
| 7 | 6,4 | 16 | 0,8 | 9,0 | 49,6 |

Die erfindungsgemäßen Verbindungen bewirken - wie aus der Tabelle ersichtlich ist - eine befriedigende bis hervorragende Anreicherung von $WO_3$ im Konzentrat.

**Patentansprüche**

1.   Bicyclische Decandicarbonsäuren erhältlich durch
a) säurekatalysierte Umsetzung von Dicyclopentadien mit einer Verbindung der Formel **(I)**,

**R-OH     (I)**

in der R aus der Gruppe der
i) linearen oder verzweigten, gesättigten Alkylreste mit 1 bis 22 Kohlenstoffatomen,
ii) linearen oder verzweigten, gesättigten Acylreste mit 2 bis 34 Kohlenstoffatomen und
iii) Reste polyalkoxylierter Fettalkohole der Formel **(II)**,

**$R^1O$-($CH_2CHR^2O$)$_n$-$CH_2CHR^2$-     (II)**

in der $R^1$ ein gesättigter Fettalkoholrest mit 2 bis 22 Kohlenstoffatomen, $R^2$ Wasserstoff und/oder Methyl und n eine Zahl von 0 bis 24 bedeuten,
ausgewählt ist, und
b) oxidative Spaltung der erhaltenen bicyclischen Verbindung an der Doppelbindung des Fünfrings.

2.   Verfahren zur Herstellung von bicyclischen Decandicarbonsäuren, **dadurch gekennzeichnet,** daß man Dicyclopentadien mit einer Verbindung der Formel **(I)**,

**R-OH     (I)**

in der R aus der Gruppe der
i) linearen oder verzweigten, gesättigten Alkylreste mit 1 bis 22 Kohlenstoffatomen,

ii) linearen oder verzweigten, gesättigten Acylreste mit 2 bis 34 Kohlenstoffatomen und
iii) Reste polyalkoxylierter Fettalkohole der Formel **(II)**,

$$R^1O\text{-}(CH_2CHR^2O)_n\text{-}CH_2CHR^2\text{-} \qquad \textbf{(II)}$$

in der $R^1$ ein gesättigter Fettalkoholrest mit 2 bis 22 Kohlenstoffatomen, $R^2$ Wasserstoff und/oder Methyl und n eine Zahl von 0 bis 24 bedeuten,
ausgewählt ist, in Gegenwart von Säuren umsetzt und die erhaltene bicyclische Verbindung an der Doppelbindung des Fünfrings oxidativ spaltet.

3. Verfahren nach Anspruch 2, **dadurch gekennzeichnet,** daß man die oxidative Spaltung mit Ozon in an sich bekannter Weise durchführt.

4. Verfahren nach Anspruch 2, **dadurch gekennzeichnet,** daß man die oxidative Spaltung mit Kaliumpermanganat in an sich bekannter Weise durchführt.

5. Verwendung von bicyclischen Decandicarbonsäuren nach Anspruch 1 als Flotationshilfsmittel.

**Claims**

1. Bicyclic decanedicarboxylic acids obtainable by
   a) acid-catalyzed reaction of dicyclopentadiene with a compound corresponding to formula (I)

   $$R\text{-}OH \qquad (I)$$

   in which R is selected from the group consisting of
   i) linear or branched, saturated $C_{1-22}$ alkyl radicals,
   ii) linear or branched, saturated $C_{2-34}$ acyl radicals and
   iii) residues of polyalkoxylated fatty alcohols corresponding to formula (II)

   $$R^1O\text{-}(CH_2CHR^2O)_n\text{-}CH_2CHR^2\text{-} \qquad (II)$$

   in which $R^1$ is a saturated $C_{2-22}$ fatty alcohol radical, $R^2$ is hydrogen and/or methyl and n is a number of 0 to 24,
   and
   b) oxidative cleavage of the bicyclic compound obtained at the double bond of the five-membered ring.

2. A process for the production of bicyclic decanedicarboxylic acids, characterized in that dicyclopentadiene is reacted with a compound corresponding to formula (I)

   $$R\text{-}OH \qquad (I)$$

   in which R is selected from the group consisting of
   i) linear or branched, saturated $C_{1-22}$ alkyl radicals,
   ii) linear or branched, saturated $C_{2-34}$ acyl radicals and
   iii) residues of polyalkoxylated fatty alcohols corresponding to formula (II)

   $$R^1O\text{-}(CH_2CHR^2O)_n\text{-}CH_2CHR^2\text{-} \qquad (II)$$

   in which $R^1$ is a saturated $C_{2-22}$ fatty alcohol radical, $R^2$ is hydrogen and/or methyl and n is a number of 0 to 24,
   in the presence of acids and the bicyclic compound obtained is subjected to oxidative cleavage at the double bond of the five-membered ring.

3. A process as claimed in claim 2, characterized in that the oxidative cleavage is carried out with ozone by methods known per se.

7

**4.** A process as claimed in claim 2, characterized in that the oxidative cleavage is carried out with potassium permanganate by methods known per se.

**5.** The use of the bicyclic decanedicarboxylic acids claimed in claim 1 as flotation aids.

**Revendications**

**1.** Acides décanedicarboxyliques bicycliques pouvant être obtenus

a) en faisant réagir en présence de catalyseurs acides du dicyclopentadiène avec un composé de formule (I),

R-OH     (I)

dans lequel R est choisi dans le groupe comportant
i) des restes alkyle saturés, à chaîne linéaire ou ramifiée, avec 1 à 22 atomes de carbone,
ii) des restes acyle saturés, à chaîne linéaire ou ramifiée, avec 2 à 34 atomes de carbone,
iii) des restes d'alcools gras polyalcoxylés de formule (II)

$R^1O\text{-}(CH_2CHR^2O)_n\text{-}CH_2CHR^{2-}$     (II)

dans laquelle $R^1$ signifie un reste d'alcool gras saturé avec 2 à 22 atomes de carbone, $R^2$ signifie l'hydrogène et/ou un reste méthyle et n signifie un nombre de 0 à 24,
et
b) scission par oxydation du composé bicyclique obtenu, à la double liaison du cycle à cinq chaînons.

**2.** Procédé pour la fabrication d'acides décanedicarboxyliques bicycliques, caractérisé en ce que l'on fait réagir le dicyclopentadiène en présence d'acides avec un composé de formule (I)

R-OH     (I)

dans lequel R est choisi dans le groupe comportant
i) des restes alkyle saturés, à chaîne linéaire ou ramifiée, avec 1 à 22 atomes de carbone,
ii) des restes acyle saturés, à chaîne linéaire ou ramifiée, avec 2 à 34 atomes de carbone,
iii) des restes d'alcools gras polyalcoxylés de formule (II)

$R^1O\text{-}(CH_2CHR^2O)_n\text{-}CH_2CHR^{2}\text{-}$     (II)

dans laquelle $R^1$ signifie un reste d'alcool gras saturé avec 2 à 22 atomes de carbone, $R^2$ signifie l'hydrogène et/ou un reste méthyle et n signifie un nombre de 0 à 24,
et qu'on scinde le composé bicyclique par oxydation, à l'endroit de la double liaison du cycle à cinq chaînons.

**3.** Procédé selon l'une des revendications 2 à 3, caractérisé en ce que la scission par oxydation est effectuée de manière connue en soi avec de l'ozone.

**4.** Procédé selon l'une des revendications 2 à 4, caractérisé en ce que la scission par oxydation est effectuée de manière connue en soi avec du permanganate de potassium.

**5.** Utilisation des acides décanedicarboxyliques bicycliques selon la revendication 1 comme adjuvants de flottation.

8